# EUROPÄISCHE PATENTSCHRIFT

(11) **EP 2 678 312 B1**
(45) Veröffentlichungstag und Bekanntmachung des Hinweises auf die Patenterteilung: **11.10.2017**
(21) Anmeldenummer: 12704512.8
(22) Anmeldetag: 13.02.2012
(51) Int. Cl.: C07C 319/20, C07C 323/25

(54) **VERFAHREN ZUR HERSTELLUNG VON 2-HYDROXY-4-(METHYLTHIO)BUTTERSÄURENITRIL AUS 3-(METHYLTHIO)PROPANAL UND CYANWASSERSTOFF**
METHOD FOR PRODUCING 2-HYDROXY-4-(METHYLTHIO)BUTANENITRILE FROM 3-(METHYLTHIO)PROPANAL AND HYDROGEN CYANIDE
PROCÉDÉ DE PRÉPARATION DE NITRILE 2-HYDROXY-4-(MÉTHYLTHIO)BUTYRIQUE À PARTIR DE 3-(MÉTHYLTHIO)PROPANAL ET DE CYANURE D'HYDROGÈNE

(30) Priorität: 23.02.2011 US 201161445781 P
(43) Veröffentlichungstag der Anmeldung: 01.01.2014
(73) Patentinhaber: Evonik Degussa GmbH, 45128 Essen (DE)
(72) Erfinder: BUSS, Dieter, 50389 Wesseling (DE); STEURENTHALER, Martin, 60320 Frankfurt (DE); RINNER, Michael, R., SG-098417 Singapore (SG); KRETZ, Stephan, 63599 Biebergemünd (DE); HASSELBACH, Hans Joachim, 63571 Gelnhausen (DE); FINKELDEI, Caspar-Heinrich, 63755 Alzenau (DE); KÖRFER, Martin, 63796 Kahl (DE); ZACCHI, Pablo, 50354 Hürth (DE)
(86) Internationale Anmeldenummer: PCT/EP2012/052367
(87) Internationale Veröffentlichungsnummer: WO 2012/113664

(56) Entgegenhaltungen:
- US-A- 4 912 257
- US-A- 5 756 803

## Beschreibung

Gegenstand der Erfindung ist ein katalytisches Verfahren zur Herstellung von 2-Hydroxy-4-(methylthio)buttersäurenitril (MMP-CN) aus 3-(Methylthio)propanal (= Methylmercapto-propionaldehyd, MMP) und Cyanwasserstoff (HCN). Insbesondere beschreibt die Erfindung einen Prozess zur Darstellung von lagerstabilem MMP-CN unter Verwendung stöchiometrischer Blausäuremengen, wobei das Produkt im Verhältnis zum nicht umgesetzten bzw. zum im Gleichgewicht zum MMP-CN stehenden MMP überstöchiometrische Mengen an Blausäure enthält.

2-Hydroxy-4-(methylthio)buttersäurenitril (MMP-Cyanhydrin) ist ein Intermediat für die Darstellung von D,L-Methionin und dem Methionin-Hydroxy-Analogen 2-Hydroxy-4-methylthio-buttersäure (MHA). Methionin ist eine essentielle Aminosäure, die u.a. als Ergänzung in Futtermitteln eingesetzt wird. MHA ist ein flüssiger Methioninersatzstoff mit geringerer biologischer Verfügbarkeit.

### Stand der Technik

Aus MMP lässt sich durch Reaktion mit Cyanwasserstoff (Blausäure) unter Verwendung geeigneter Katalysatoren das MMP-Cyanhydrin (2-Hydroxy-4-(methylthio)buttersäurenitril) herstellen. Geeignete Katalysatoren sind z.B. Pyridin oder Triethylamin. Durch Hydrolyse von MMP-Cyanhydrin mit z.B. Mineralsäuren erhält man MHA. Methionin entsteht durch Reaktion von MMP-Cyanhydrin mit Ammoniumhydrogencarbonat unter Bildung von Hydantoin, das mit einer Base, z.B. Kaliumcarbonat oder Natriumhydroxid verseift werden kann. Die Freisetzung des Methionins geschieht mit Kohlendioxid oder Schwefelsäure.

Es ist beispielsweise aus dem Patent US A 4 960 932 bekannt, Methionin nach einem vierstufigen Verfahren herzustellen. Im ersten Schritt wird durch Addition von HCN an MMP in Gegenwart von Triethylamin das MMP-Cyanhydrin hergestellt. Die eingesetzte Menge an HCN entspricht 1,05 Mol in Bezug zur eingesetzten Menge an MMP. Dann wird das MMP-Cyanhydrin in einem zweiten Schritt mit Ammoniak umgesetzt, wodurch das 2-Amino- 4-methylthiobutyronitril gebildet wird, das dann in einem dritten Schritt in Gegenwart eines Ketons und eines Alkalihydroxids unter Bildung des Methylthiobutyramids hydrolysiert wird, das schließlich zu einem Alkalimethioninat verseift wird.

Im Falle der Herstellung von 2-Hydroxy-4-methylthiobuttersäure (MHA) wird das 2-Hydroxy-4-methylthio-butyronitril durch Umsetzung von MMP und HCN in einem Medium erhalten, das Pyridin oder ein Amin enthält (siehe Patent US A 2 745 745, Spalte 2, Zeilen 52 bis 55). Überschüssiges HCN wird lediglich abdestilliert, z.B. im Vakuum. Das erhaltene 2-Hydroxy-4-methylthio-butyronitril wird dann mit Schwefelsäure hydrolysiert, wodurch unmittelbar das Amid von 2-Hydroxy-4-methylthiobuttersäure und schließlich die 2-Hydroxy-4-methylthiobuttersäure gebildet wird. Ein ähnliches Verfahren ist auch in EP A 330 527 A1 bzw. in US 4 912 257 beschrieben.

Weiterhin wird in WO 96/40631 A1 die Herstellung von MMP-Cyanhydrin durch die Umsetzung von MMP mit Cyanwasserstoff in Gegenwart eines geeigneten Additionsreaktionskatalysators beschrieben. Dort wurde gefunden, dass Triisopropanolamin, Nicotinamid, Imidazol, Benzimidazol, 2-Fluorpyridin, Poly-4-vinylpyridin, 4-Dimethylaminopyridin, Picolin und Pyrazin als Additionsreaktionskatalysator zur Herstellung von MMP-Cyanhydrin dienen können. Ferner können auch Trialkylamine mit drei bis achtzehn Kohlenstoffatomen in jedem der an das Stickstoffatom gebundenen Alkylsubstituenten und tertiäre Amine, in denen wenigstens einer der an das Stickstoffatom gebundenen Nicht-Wasserstoff-Substituenten gemäß obiger Beschreibung eine Arylgruppe enthält, dazu dienen, die Reaktion zwischen MMP und Cyanwasserstoff zu MMP-Cyanhydrin zu katalysieren.

Vorzugsweise wird dabei der Cyanwasserstoff in einem molaren Überschuss von etwa 2 % bezogen auf MMP eingesetzt.

WO 2006/015684 A2 offenbart schließlich ein Verfahren zur insbesondere kontinuierlichen Herstellung von MMP bzw. von MMP-Cyanhydrin, bei dem jeweils heterogene AminKatalysatoren für die Additionsreaktion verwendet werden.

Weiterhin ist aus dem Patent US 5 756 803 bekannt, einen Aldehyd mit Cyanwasserstoff in Gegenwart eines Puffers, mit dem der pH-Wert der Lösung über 4 eingestellt werden kann, umzusetzen, wobei Amine ausgenommen sind. Ganz allgemein können als Puffer Gemische von Alkalisalzen von Säuren und Säuren oder Gemische von Säuren und Alkalihydroxiden verwendet werden. Der Puffer wird verwendet, um einerseits die Zersetzung der Ausgangsstoffe und des gewünschten Produktes zu vermeiden und andererseits die zur Stabilisierung von Cyanwasserstoff verwendeten Säuren zu neutralisieren. Ebenfalls wird hier HCN in molarem Überschuss zum MMP dosiert, wobei der molare Überschuss vorzugsweise im Bereich von 2 % bis 5 % liegt. Bei der Umsetzung des MMP mit HCN in Gegenwart der üblicherweise verwendeten Basen erhöhen diese unter den angegebenen Bedingungen zwar die Reaktionsgeschwindigkeit, führen jedoch schnell zu einer Zersetzung des gebildeten Cyanhydrins und einer Zersetzung des anfänglich eingesetzten Aldehyds unter Bildung einer stark gefärbten Lösung.

Um die im Abgas des Reaktivabsorbers enthaltenen Restmengen an nicht umgesetzter HCN und MMP zurückzugewinnen, werden diese mittels eines Wasserwäschers ausgewaschen, wobei das Waschwasser in das Produkt gelangt. Der Wassergehalt im Produkt beträgt ca. 48 Gew-%.

Die wesentlichen Nachteile der bisher in der Literatur beschriebenen Verfahren bestehen darin, dass zur Erreichung einer hohen MMP-CN-Ausbeute hohe molare Überschüsse an HCN eingesetzt werden müssen. Die überschüssigen Mengen an HCN gehen in den beschriebenen Verfahren verloren und bedeuten einen großen wirtschaftlichen Nachteil. Weiterhin fördern die eingesetzten Katalysatoren in den beschriebenen Verfahren auch die Bildung von unerwünschten Nebenprodukten aus den eingesetzten Aldehyden, die zu einer nicht tolerierbaren Verunreinigung des Produktes führen. Ein Ansatz zur Lösung dieses Problems der Bildung von Nebenprodukten wird in US 5 756 803 beschrieben, wobei allerdings große Mengen an Wasser in das Produkt gelangen, die einerseits zur Herstellung von Methionin wenigstens teilweise wieder entfernt werden müssen und die andererseits wiederum die Zersetzung des MMP-Cyanhydrins begünstigen, was jeweils einen nicht unerheblichen Nachteil bedeutet. Daher ist das in dem Patent US 5 756 803 beschriebene Produkt nicht lagerstabil und muss zur Lagerung und insbesondere für den Transport aufwändig mittels destillativer Abtrennung des Wassers aufgearbeitet werden, was einen hohen wirtschaftlichen Nachteil des Verfahrens bedeutet.

### Aufgabe der Erfindung

Es war Aufgabe dieser Erfindung, ein katalytisches Verfahren bereitzustellen, das sowohl die Reaktion von Aldehyd, insbesondere von MMP mit Cyanwasserstoff katalysiert, als auch ein lagerstabiles Cyanhydrin bereitstellt, wobei das Verfahren gleichzeitig deutliche Verbesserungen hinsichtlich der Ausbeuten bzgl. des eingesetzten Aldehyds und des Cyanwasserstoffs aufweisen sollte.

### Beschreibung der Erfindung

Diese und weitere Aufgaben werden gelöst durch ein Verfahren zur Herstellung von 2-Hydroxy-4-(methylthio)-buttersäurenitril, bei dem 3-(Methylmercapto)-propionaldehyd mit Cyanwasserstoff in Gegenwart einer Base als Katalysator in einer Hauptreaktionszone zum Nitril umgesetzt wird und Restmengen an gasförmigem Cyanwasserstoff (HCN), welche die Hauptreaktionszone verlassen in einer Absorptions- und Nachreaktionszone enthaltend eine Mischung aus 3-(Methylmercapto)-propionaldehyd und Katalysator und wahlweise 2-Hydroxy-4-(methylthio) buttersäurenitril absorbiert und weiter umgesetzt werden.

Der Restgehalt von HCN reagiert infolge der Absorption bzw. Kondensation mit dem Aldehyd zum Cyanhydrin. Aufgrund der effektiven Entfernung des HCN aus der Gasphase ist es möglich, im Unterschied zu den in der Literatur bekannten Verfahren ein Molverhältnis von Cyanwasserstoff zu Aldehyd von 0,99 bis 1,01 zu verwenden, was einen großen wirtschaftlichen Vorteil für das Verfahren bedeutet.

Die Erfindung bezieht sich insbesondere auf ein Verfahren zur Additionsreaktion von Cyanwasserstoff an MMP in Gegenwart einer Base, insbeondere eines Amins, wobei das Verfahren derart ausgestaltet ist, dass Restgehalte an gasförmigem Cyanwasserstoff außerhalb einer Hauptreaktionszone bei Temperaturen von etwa 0 °C bis 25 °C in eine flüssigen Mischung aus dem Aldehyd MMP und dem Reaktionsprodukt aus MMP mit Cyanwasserstoff und Katalysator absorbiert und dann mit MMP weiterumgesetzt werden.

Mit dem erfindungsgemäßen Verfahren können generell Aldehyde enthaltend 1 bis 6 Kohlenstoffatome, die gegebenenfalls mit Alkyl, Alkoxy oder Alkylthio substituiert sind, vorteilhaft mit Cyanwasserstoff umgesetzt werden.

Dabei bevorzugt ist, dass die in der Absorptions- und Nachreaktionszone enthaltene Mischung zumindest teilweise aus der Hauptreaktionszone stammt. Dadurch wird im Gegensatz zu US 5,756,803 eine Verdünnung mit Fremdstoffen oder -Lösemitteln verhindert.

Die Hauptreaktionszone kann sowohl einen Rührreaktor oder einen Schlaufenreaktor beinhalten. Beide Ausführungsformen führen zu einer schnellen und guten Durchmischung und einem schnellen Umsatz von MMP und HCN.

Die Hauptreaktionszone kann auch zusätzlich eine Strahlpumpe beinhalten. Dies führt zu einer weiteren Intensivierung der Mischung der Komponenten und kann besonders vorteilhaft zum Einsaugen von HCN in die Hauptreaktionszone mit verwendet werden.

Die Nachreaktion kann wie oben angegeben zwischen einem HCN-haltigen Gas und einer Flüssigkeit erfolgen. Sie findet dann in einer Absorptions- und Nachreaktionszone statt, die vorzugsweise eine Vorrichtung zum In-Kontakt-Bringen eines Gases mit einer Flüssigkeit beinhaltet, insbesondere eine Kolonne wie beispielsweise eine Bodenkolonne, eine Füllkörperkolonne, einen Blasensäulenreaktor, eine Tropfenkolonne oder wahlweise einen Reaktor mit mechanisch gerührtem Behälter oder einem Tauchstrahlreaktor.

Die Absorptionszone und die Nachreaktionszone kann auch Teil eines Schlaufenreaktors sein, was eine hohe Vermischung und schnelle Umsetzung der Komponenten bewirkt.

Beim erfindungsgemäßen Verfahren wird im Wesentlichen gasförmiger Cyanwasserstoff in die Hauptreaktionszone eingebracht, vorzugsweise ein cyanwasserstoffhaltiges Produktgas aus einem Cyanwasserstoffproduktionsverfahren.

Der Cyanwasserstoff-Gehalt des eingesetzten Gasgemisches reicht von 1 bis 99 Gew.%, vorzugsweise von 5 bis 75 Gew.%, besonders bevorzugt von 6-22 Gew.%. Der Cyanwasserstoff wird insbesondere nach dem Andrussow-Verfahren gemäß DE 102007034715A1 oder auch nach dem sogenannten BMA-Verfahren (Blausäure aus Methan und Ammonik) gemäß DE1041476 (Reaktor) hergestellt. Beide Verfahren sind auch beschrieben in Ullmann's Encyclopedia of Industrial Chemistry, 1987 VCH-Verlagsgesellschaft mbH,Kapitel "Cyano Compounds Inorganic", Abschnitt 1.2.1 -1.2.2. Der enthaltene Ammoniak wird jeweils aus dem Produktgas entfernt. Das Produktgas aus dem Andrussow-Verfahren (Andrussow-Gas) enthält nach der Ammoniakabtrennung typischerweise etwa 10 Gew.% Cyanwasserstoff, das Produktgas aus dem BMA-Verfahren (BMA-Gas) dagegen etwa 70 Gew.% Cyanwasserstoff.

So weisen die typischen Produktgaszusammensetzungen des Andrussow-Verfahrens etwa folgende Gehalte auf: 10,3 Gew.% HCN, 3,7 Gew. % H₂O 1,3 Ges.% H₂, 75,8 Gew.% N₂, 0,4 Gew.% O₂, 6, 3 Gew. % CO, 0,6 Gew. % CO₂, 0,4 Gew. % CH₄, 1, 3 Gew.% Ar, die des BMA-Verfahrens etwa 68,3 Gew.% HCN, 6,7 Gew.% H₂O, 17,3 Gew. % H₂, 3,6 Gew.% N₂, 4 Gew. % CH₄.

Die direkte Verwendung des Produktgases hat den erheblichen Vorteil, dass keine vorgeschaltete und energieintensive Verflüssigung des Cyanwasserstoffs erfolgen muss und bei entsprechender Kopplung mit einer Anlage zur Herstellung von Cyanwasserstoffgas erhebliche Investitionen in entsprechende Verfahrensstufen zur Absorption und Destillation des HCN eingespart werden. Die weiteren Gasanteile neben HCN haben überraschenderweise keinen nachteiligen Einfluss auf die Cyanhydrin-Ausbeute.

Das Restgas der MMP-Cyanhydrin- und der CyanwasserstoffHerstellung kann anschließend gemeinsam verwertet oder verbrannt werden. Im letzten Fall kann die dabei gewonnene Energie für den Betrieb beider Verfahren wiederverwendet werden, was mehr Freiheitsgrade und einen erheblichen wirtschaftlichen Vorteil bedeutet.

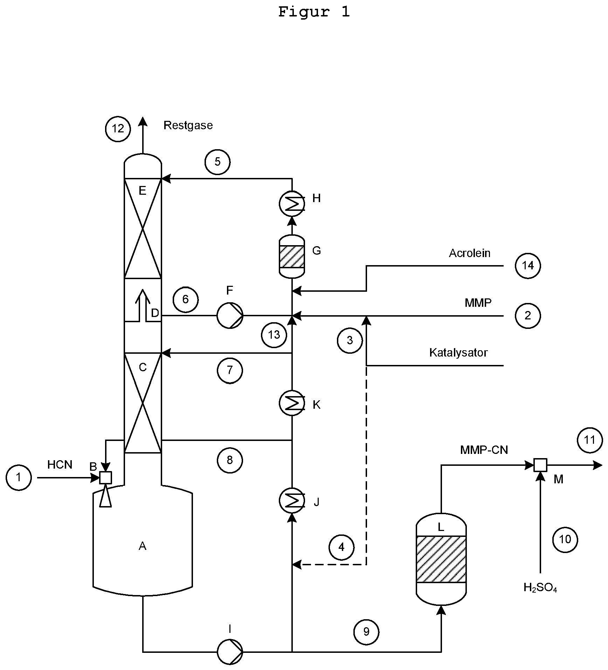

Eine bevorzugte Ausführungsform des erfindungsgemäßen Verfahrens bzw. einer entsprechenden Vorrichtung ist in **Fig. 1** skizziert, welche nachstehend näher erläutert wird:
Bei Verwendung einer Bodenkolonne oder einer Füllkörperkolonne zur reaktiven Absorption wird der Gasfluss, der die Blausäure enthält, in den Sumpf (A) der Kolonne (C) zugeführt oder vorzugsweise bereits über einen Gasventilator (B) mit der Aldehydlösung in Kontakt gebracht, die mittels einer Pumpe (I) im Kreis (8) gefördert wird. Die Temperatureinstellung im Sumpf der Kolonne erfolgt mittels Wärmeübertrager (J). Insbesondere der Sumpf (A) und die Kolonne (C) dienen als Hauptreaktionszone, wobei die Kolonne C gesondert mittels Wärmeübertrager (K) temperiert werden kann. Dabei wird die Temperatur der Ströme (7) und (8) so gewählt, dass die Reaktionswärme mit Kühlwasser entsprechend der Umgebungstemperatur abgegeben werden kann und die Reaktion zwischen Aldehyd und HCN im Kolonnenteil (C) zu 80 % bis 99,9 % abgeschlossen ist.

Die Zufuhr des Aldehyds kann getrennt oder zusammen mit dem Katalysator erfolgen((2),(3)). Vorzugsweise wird der Aldehyd oder die Aldehyd / Katalysatormischung (2) + (3) mit einem Teilstrom (6) aus dem Absorptions- und Kondensationsteil (E) der Kolonne, der einem Zwischensumpf (D) entnommen wird, vermischt. Die Zufuhr des Katalysators kann z.B. auch über den Weg (4) erfolgen. In diesem Fall sollte der Katalysator über den Weg (13) anteilig auch in den Kopfkreislauf gelangen. Die im Strom (6) enthaltenen Restmengen an HCN werden mit dem zugeführten Aldehyd im Verweilzeitbehälter (G), der (zweiten) Nachreaktionszone, vollständig bzw. nahezu vollständig zum Cyanhydrin umgesetzt. Danach wird der Strom im Wärmetauscher (H) auf 0 °C bis 25 °C abgekühlt, um die möglichst vollständige Kondensation/Absorption von HCN zu gewährleisten. Insbesondere der Zwischensumpf (D), der Absorptions- und Kondensationsteil (E) und der Verweilzeitbehälter (G) dienen als Absorptions- und Nachreaktionszone. Aufgrund der im Strom (5) enthaltenen Mengen an Cyanhydrin und der erfolgten Abkühlung enthalten die am Kolonnenkopf austretenden Restgase auch nur ganz wenige Restmengen des Aldehyds, sodass es keinerlei zusätzlicher Wäsche zur Rückgewinnung des Aldehyds aus dem Restgas bedarf. Die Konzentration des Cyanhydrins kann über eine entsprechende Dosierung aus dem Kolonnensumpf (13) vorzugsweise im Bereich von 10 Gew.-% bis 70 Gew.-% im Strom (5) eingestellt werden. Die gereinigten Gase werden vorteilhaft in eine Verbrennungsanlage geleitet. Das mit Strom (9) austretende Produkt hat ein Molverhältnis von Cyanwasserstoff zu nicht umgesetztem Aldehyd von größer als 1, was zur Stabilisierung des Produktes wesentlich beiträgt. Weiterhin ist das Produkt klar und nur schwach gefärbt, was die außerordentlich hohe Selektivität dieser Verfahrensausführung unterstreicht.

Nach dem Passieren eines Nachreaktors (L), in dem gegebenenfalls enthaltene Restanteile des Aldehyds bis zum Erreichen des Gleichgewichtes mit Cyanwasserstoff abreagieren, wird der so erhaltene Produktstrom mit einer Säure vermischt. Hierzu wird ein geeignetes Mischorgan (M) verwendet. Der dabei eingestellte pH-Wert des Produktes (Strom (11)) liegt zwischen 1 und 4, bevorzugt zwischen 2 und 3.

Handelt es sich beim Aldehyd um MMP, wie in Fig.1 dargestellt, so enthält der MMP-Eduktstrom des beschriebenen Verfahrens in der Regel einen kleinen Anteil an Methylmercaptan (MC), das zum überwiegenden Teil in den Abgasstrom (12) gelangen würde. Dieses überschüssige MC kann auch wahlweise mit Acrolein, das dem Verfahren z. B. über Strom (14) zugeführt werden kann, zu MMP und in Folge mit HCN zu MMP-CN umgesetzt werden und damit die Ausbeute weiter gesteigert werden.

Beim erfindungsgemäßen Verfahren können als Katalysator niedermolekulare oder heterogene Amine oder Lösungen von anorganischen Basen oder Mischungen aus Säuren und niedermolekularen Aminen verwendet werden. Diese werden auch gebraucht, um den für die Reaktion benötigten optimalen pH-Bereich von ca. 4,5 bis 6,0, vorzugsweise 5,0 -5,5 einzustellen, der mit einer pH-Elektrode (Typ: "Aquatrode Plus mit Pt 1000", Hersteller: Metrohm Schweiz AG) direkt im Cyanhydrin mit einem typischen Wassergehalt von 2 - 14 Gew.% gemessen wird. Die Messungen erfolgen bei einer Temperatur von etwa 23° in einem gerührten Gefäß, wobei die pH-Messung temperaturkompensiert ist. Zur zeitnahen Nachverfolgung der Reaktionsbedingungen und zur Eliminierung von Messfehlern erfolgt im Abstand einer Stunde jeweils eine 4-fach-Messung des pH-Wertes mit Bildung des Mittelwertes, wobei jede Messung ungefähr 30 Sekunden lang dauert. Die Messung kann aber auch direkt während der Reaktion online im Reaktions-system bei der dort eingestellten Temperatur durchgeführt werden und auf den pH-Wert bei 23 °C umgerechnet werden, was die Verfahrenskontrolle weiter vereinfacht.

Niedermolekulare Amine, vorzugsweise mit 1 bis 36 C-Atomen, haben den besonderen Vorteil der praktisch unbegrenzten Mischbarkeit mit dem Reaktionsmedium, was wiederum eine schnelle Reaktion begünstigt.

Dabei bevorzugte niedermolekulare Amine sind Tri-(C₁-C₁₂-alkyl)-amine, vorzugsweise Triethylamin oder Triisopropanolamin, Dialkylaralkylamine, vorzugsweise Dimethylbenzylamin, Dialkylarylamine, vorzugsweise N,N-Dimethylanilin, heterozyklische Amine, vorzugsweise Nicotinamid, Imidazol, Benzimidazol, 2-Fluorpyridin, 4-Dimethylaminopyridin, Picolin oder Pyrazin.

Alternativ können auch heterogene Amine der allgemeinen Formel 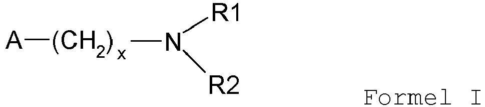 oder Polyvinylpyridin verwendet werden, wobei
R₁ und R₂ Wasserstoff, Alkyl mit Kettenlängen zwischen C₁ und C₁₂, Aryl oder Heteroaryl sind;
R₁ kann verschieden von R₂ sein
X ist eine Zahl zwischen 0 und 6, und
A ist ein natürliches oder synthetisches Harz, vorzugsweise ein Polystyrol. Diese und die damit verbundenen Vorteile wie etwa leichtere Abtrennbarkeit, geringe Verschleppung in nachfolgende Reaktionsstufen sind bereits in der WO 2006/015684 beschrieben.

Dabei bevorzugt ist, dass der Katalysator gemäß Formel I eine Polymer-gebundene Base ausgewählt aus der Gruppe der homologen Dialkylaminoalkylpolystyrole oder Dialkylaminomakroretikulären Harze ist.

Besonders bevorzugt wird, dass der Katalysator gemäß Formel I Diethylaminoethylpolystyrol, Diethylaminomethylpolystyrol, Dimethylaminomethylpolystyrol, Diethylaminomethylmakroretikuläres Harz oder Dimethylaminoethylpolystyrol ist.

Als anorganische Base können vorteilhafterweise Alkalihydroxid, vorzugsweise NaOH oder KOH, Alkalicyanid, vorzugsweise NaCN oder KCN, Alkalicarbonat, vorzugsweise Na₂CO₃ oder K₂CO₃, oder Alkalihydrogencarbonat, vorzugsweise NaHCO₃ oder KHCO₃, alleine oder in gemischter Form verwendet werden. Diese haben den Vorteil der besonders hohen katalytischen Wirkung, was wiederum eine sehr schnelle Reaktion begünstigt sowie des geringen Störpotentials der daraus entstehenden geringen Salzanteile im nachfolgenden Verfahren. Allerdings muß hier für extrem gute Durchmischung und Temperaturkontrolle gesorgt werden, damit keine nennenswerte Nebenproduktbildung erfolgt.

Als Katalysatoren können in vorteilhafter Weise auch Mischungen aus Säuren und den oben genannten niedermolekularen Aminen eingesetzt werden, um den pH-Wert im gewünschten Bereich besser einstellen und durch die Pufferwirkung stabilisieren zu können. Besonders vorteilhaft hierbei ist die Verwendung von organischen Säuren wie kurzkettigen Fettsäuren z.B. Essigsäure, Ameisensäure, Zitronensäure, und organische Sulfonsäuren, z. B. Trifluormethansulfonsäure oder die Verwendung von Mineralsäuren, wie z.B. Schwefelsäure oder Phosphorsäure in Verbindung mit den niedermolekularen Aminen.

Nach einer weiteren bevorzugten Ausführungsform der Erfindung wird die Temperatur in der Hauptreaktionszone so gewählt, dass die frei werdende Rektionswärme an Kühlwasser entsprechend der Umgebungstemperatur abgegeben werden kann, was einen weiteren großen wirtschaftlichen Vorteil des Verfahrens bedeutet.

Entsprechend wird die Hauptreaktionszone bei einer Temperatur von 20°C bis 80°C, vorzugsweise von 30°C bis 70°C , besonders bevorzugt von 35°C bis 65°C betrieben. In diesem Bereich verläuft die Reaktion auch vergleichsweise schnell.

Beim erfindungsgemäßen Verfahren ist weiter bevorzugt, dass die Absorptions- und Nachreaktionszone bei einer Temperatur von 0°C bis 30°C, vorzugsweise von 4°C bis 15°C betrieben wird. Dies sorgt für eine besonders effiziente Absorption des Cyanwasserstoffs und ermöglicht immer noch eine weitgehende Umsetzung von HCN mit dem MMP zum MMP-Cyanhydrin.

Außerdem vorteilhaft ist die Verwendung einer zweiten Nachreaktionszone kurz vor der Produktaustragsstelle des MMP-Cyanhydrins. Diese wird bei einer ähnlichen Temperatur wie die Hauptreaktionszone von 20°C bis 80°C, vorzugsweise von 40°C bis 70°C, besonders bevorzugt von 45°C bis 65°C betrieben. So wird eine schnelle und praktisch quantitative Vervollständigung der Umsetzung von HCN und MMP zum MMP-Cyanhydrin kurz vor dem Produktaustrag gewährleistet.

Das erfindungsgemäße Verfahren wird vorteilhafterweise unter einem Absolutdruck von 0,9 bis 5 bar, vorzugsweise von 1,0 bis 3 bar, besonders bevorzugt von 1 bis 1,5 bar betrieben. Dies hat den Effekt, dass ein schnelles Ausgasen des absorbierten HCN aus der Lösung und damit entsprechende Verluste verhindert werden.

Das erfindungsgemäße Verfahren ist ferner dadurch gekennzeichnet, dass ein molares Verhältnis von Blausäure zu 3-(Methylthio)propanal von 0,98 bis 1,03, bevorzugt von 0,99 bis 1,01 eingestellt werden kann. Zum einen werden dadurch Verluste von Blausäure vermieden, die gerade im großtechnischen Maßstab von großem wirtschaftlichen Nachteil sind. Zum anderen werden unerwünschte Blausäureabbauprodukte wie z.B. polymere Blausäure oder das Verseifungsprodukt Ameisensäure, welches korrosive Eigenschaften gegenüber diversen metallischen Werkstoffen hat, vermieden und damit entsprechende nachteilige Wirkungen in den nachgeschalteten Verfahrensstufen zum Methionin.

Beim erfindungsgemäßen Verfahren wird vorzugsweise ein Gewichtsverhältnis von Katalysator zu 3-(Methylthio)propanal von 0,00005 bis 0,002, besonders bevorzugt von 0,0001 bis 0,001 verwendet. Dies hat den Effekt einer besonders geringen Nebenproduktbildung bei gleichzeitig hoher Reaktionsgeschwindigkeit.

Das erfindungsgemäße Verfahren kann wahlweise im Batch, halbkontinuierlich oder auch kontinuierlich durchgeführt wird, wobei die kontinuierliche Ausführungsform im großtechnischen Maßstab von über 10,000 tons/a besonders wirtschaftlich zu betreiben ist.

Das erfindungsgemäß hergestellte MMP-Cyanhydrin hat typischerweise folgende Zusammensetzung:
MMP-CN: 86 - 97 Gew%,
MMP: 0 - 1 Gew%,
HCN: 0,05 - 0,5 Gew%,
H₂O: 2 - 14 Gew%,
Oligomere: 0,01 - 0,1 Gew%.

Die molaren Ausbeuten bezogen auf MMP betragen typischerweise 99,50 bis 99,99 %.

Das nach einem erfindungsgemäßen Verfahren erhaltene Methylthiopropionaldehydcyanhydrin (MMP-Cyanhydrin) enthaltende Reaktionsprodukt kann in besonders vorteilhafter Weise direkt verwendet werden zur Herstellung von Methionin und von 2-Hydroxy-4-methylthiobuttersäure. Dazu wird es entweder aminiert (Aminonitrilweg) oder mit einem Gemisch von Ammoniak und Kohlendioxid umgesetzt (Hydantoinweg), um das Methionin zu bilden oder direkt zu 2-Hydroxy-4-methylthiobuttersäure (Methioninhydroxyanaloges, MHA) hydrolysiert. Es wurde darüber hinaus überraschenderweise gefunden, dass in MMP bereits vorhandene hochsiedende MMP-Oligomere im erfindungsgemäßen Verfahren zum überwiegenden Teil zum gewünschten MMP-Cyanhydrin umgesetzt werden. Dies zeigt sich z.B. daran, dass der bei der Destillation der Produkte entstehende Rückstand nach der Reaktion deutlich geringer ausfällt als vor der Reaktion zum MMP-Cyanhydrin.

Die vorliegende Erfindung wird anhand der folgenden Beispiele näher erläutert.

### Verwendete Analysenmethoden:

Der Gehalt an H₂O in MMP-CN wurde nach der Methode der Titration mit biamperometrischer Indikation des Endpunktes (Karl-Fischer-Titration) bestimmt.

Hierzu wurden im Titriergefäß 20-30 ml Titriermedium (z.B. Hydranal Solvent 5, Fa. Fluka) vorgelegt und mit Titriermittel (z.B. Hydranal Titrant 5, Fa. Fluka) trockentitriert. Eine Probenmenge von ca. 500 mg wurde der austitrierten Vorlage zugesetzt (Kunststoffeinwegspritze) und mit Titriermittel bis zum Endpunkt titriert. Die Bestimmung der genauen Probeneinwaage erfolgte durch Differenzwägung.

Die Durchführung dieser Standardmethode ist dem Fachmann bekannt (siehe z.B. P. A. Bruttel, R. Schlink: *Wasserbestimmung durch Karl-Fischer-Titration* Metrohm AG).

Die Bestimmung des Produktgehaltes an freier Blausäure erfolgte nach dem Prinzip der Ionenchromatographie (IC) mit amperometrischer Cyaniddetektion an einer Ag-Arbeitselektrode, wobei als Probenvorbereitung eine Abtrennung der freien Blausäure von der Probenmatrix mittels präparativer Säulenchromatographie erfolgte.

Die Durchführung der präparativen Cyanidabtrennung erfolgte z. B. bei Raumtemperatur an einer PRP-X 300 - Trennsäule, 250 mm Länge x 4,1 mm Innendurchmesser der Fa. Hamilton. Die mobile Phase bestand aus einer 5 mmolaren Schwefelsäure. Bei einem Fluss von 1,0 ml/min wurden 100 µl der Probelösung (0,1 g Probe in 10 ml mobiler Phase) injiziert. Das Säuleneluat von 4 min bis 8 min wurde in einem 100 ml-Messkolben gesammelt, mit Reinstwasser zur Marke aufgefüllt und 100 µl in den IC zur Cyanid-Bestimmung injiziert.

Analog der Probelösung wurde eine NaCN-Kalibrierlösung bekannten Gehaltes der präparativen Abtrennung mittels Säulenchromatographie unterzogen und 100 µl in den IC zur Cyanidbestimmung injiziert.

Die Durchführung der ionenchromatographischen Cyanidbestimmung erfolgte bei Raumtemperatur z.B. an einer Carbo Pac PA1-Trennsäule, 250 mm Länge x 4,0 mm Innendurchmesser der Fa. Dionex. Die mobile Phase bestand aus einer Lösung von 1,5 g Natriumchlorid und 1 ml Ethylendiamin in 1 1 einer 50 mmolaren Natronlauge. Bei einem Fluss von 1,0 ml/min wurden 100 µl Probe- bzw. Kalibrierlösung injiziert. Die Auswertung erfolgte durch Peakflächenvergleich mittels der externen Standardmethode.

Die Durchführung dieser Standardmethode ist dem Fachmann bekannt.

Die Bestimmung des Produktgehaltes an MMP-CN und MMP erfolgte mittels isokratischer Ionenausschlusschromatographie an einem Kationenaustauscher mit anschließender UV-Detektion bei 205 nm. Die Durchführung der Bestimmung erfolgte z. B. an einer PRP-X 300-Trennsäule, 250 mm Länge x 4,1 mm Innendurchmesser der Fa. Hamilton bei einer Temperatur von 25 °C. Die mobile Phase bestand aus einer 5 mmolaren Schwefelsäure. Bei einem Fluss von 1,0 ml/min wurden 100 µl der jeweiligen Probelösung (0,5 g Probe für MMP-Bestimmung bzw. 0,06 g Probe für MMP-CN-Bestimmung in 50 ml Lösungsmittel) injiziert. Die Kalibrierung erfolgte durch Injektion von geeigneten Kalibrierlösungen (0,5 mg MMP in 50 ml Lösungsmittel bzw. 50 mg MMP-CN in 50 ml Lösungsmittel).

Das Lösungsmittel bestand aus einer Mischung von 500 µl 0,5 molare H₂SO₄ und 5 ml Acetonitril, die mit Reinstwasser zu 50 ml verdünnt wurde.

Die Auswertung erfolgte durch Peakflächenvergleich mittels der externen Standardmethode.

Die Durchführung dieser Standardmethode ist dem Fachmann bekannt.

Die Bestimmung der Komponenten im HCN-haltigen Eduktgas Stickstoff (N₂), Kohlenmonoxid (CO), Kohlendioxid (CO₂), Methan (CH₄), Ammoniak (NH₃), Blausäure (HCN), Wasser (H₂O) , Argon (Ar) / Sauerstoff (O₂) (entweder/oder), Wasserstoff (H₂) (nur bedingt) und Benzol als internem Standard erfolgte mittels Gaschomatographie. Verwendet wurde hierbei der Gaschromatograph 6890 (Agilent, auf Basis des HP 6890) Der Gaschromatograph war für diese Analytik mit drei Trennsäulen: 1. HP-CPWAX 52CB 25m*0,32mm*0,2µm (hier wurden NH₃, HCN, Wasser und Benzol getrennt), 2. Molsieb 30m*0,32mm*12µm (hier wurden H₂, N₂, O₂, CO und Methan getrennt) und 3. Plot Q 30m*0,32mm*20µm (hier wurden CO₂ und Benzol getrennt), zwei Wärmeleitfähigkeitsdetektoren (WLD), einer Druckmesseinheit und einem Massendurchflußmesser (MDM) für Helium ausgestattet. Die Säule 1 war über einen Back Injektor mit dem Back Detektor verbunden. Die Säulen 2 und 3 waren mit einem Front Injektor mit dem Front Detektor verbunden.

Die Durchführung dieser Standardmethode ist dem Fachmann bekannt.

Die Die Bestimmung der Komponenten Methylmercaptan (MC) und Methylmercaptopropionaldehyd (MMP) und Acrolein (AC) des im Kolonnenkopf austretenden Restgases erfolgte mittels Gaschromatographie. Verwendet wurde hierbei der Gaschromatograph 7890A (Agilent). Der Gaschromatograph war für diese Analytik mit einer Trennsäule (HP-INNOWAX 60m*0,32mm*0, 25µm) und einem Back Detektor (FID) ausgestattet. Die Durchführung dieser Standardmethode ist dem Fachmann bekannt.

### Beispiel 1

Es wurde eine Apparatur wie in Fig. 1 gezeigt mit einer Kolonne von 70 mm Durchmesser verwendet, die mit 2 Packungen (C) und (E) ausgestattet war und die eine Höhe von jeweils 2500 und 1700 mm aufwiesen. Zwischen den Packungen befand sich ein Zwischensumpf (D), dem zum Betrieb eines Kopfkreislaufs ein Strom (6) entnommen werden kann. Unterhalb der Kolonne befand sich der Kolonnensumpf mit einem Volumen von 4 Litern. Das Schema dieser Vorrichtung ist beigefügt (siehe Figur 1).

Mit dem Strom (1) wurden 8,98 kg/h rohes Produktgas der Cyanwasserstoffherstellung nach dem Andrussow-Verfahren über den Gasventilator (B) in den Kolonnensumpf A zugeführt, das auf das Gewicht bezogen enthielt: HCN: 8,87%, H₂O: 3, 88 %, H₂: 1,33 %, N₂: 76,01 %, O₂: 1,48 %, CO: 5,67 %, CO₂: 1,13 %, CH₄: 0,39 %. Das eintretende Gas wurde an der Strahlpumpe (B) mit einem umlaufenden Strom (8) von 300 kg/h vermischt. Der Umlaufstrom war dabei so temperiert, dass im Kolonnensumpf (A) bei einem Füllstand von 50% eine Temperatur von 50 °C herrschte. Der Zulaufstrom (7) auf die Packung (C) hatte bei 40 kg/h eine Temperatur von 35 °C.

Der Methylthiopropionaldehyd wurde mit einem Durchsatz von 2,966 kg/h über die Zuführung (2) in den Reaktor (G) eingeführt. Er enthielt auf das Gewicht bezogen:
MMP: 96,46 %, H₂O: 2,77 %, MC: 0,2 %. Über die Zuleitung (3) wurden gleichzeitig 0,211 kg/h eines Gemisches aus 99 Gew.% MMP in der oben beschriebenen Zusammensetzung und 1 Gew.% Triethanolamin als Katalysator in den Reaktor (G) eingeführt. Der Gesamtstrom (5) bestehend aus den Edukten und dem Kreislaufstrom (6) betrug in der Zuführung zur oberen Packung (E) 40 kg/h bei einer Temperatur von 6 °C.

Das molare Eduktverhältnis HCN/MMP entsprach 1. Das Produkt verließ den Kolonnensumpf mit 4,20 kg/h und hatte bezogen auf das Gewicht folgende Zusammensetzung: MMP-CN: 90,43 %, H₂O: 7,82 %, MMP: 0,14 %, HCN: 0,16 %, MC: 0,01 %. Das Abgas verließ den Kolonnenkopf mit 8,07 kg/h und hatte bezogen auf das Gewicht folgende Zusammensetzung: HCN: 0,00 %, MMP: 0,07 %, MC: 0,05 %, H₂O: 1,34 %, H₂: 1,48 %, N₂: 86, 02 %, O₂: 1,64 %, CO: 6,31 %, CO₂: 1,26 %, CH₄: 0,44 %. Die Gase wurden einer Verbrennungsanlage zugeführt.

### Beispiel 2

Es wurde die Apparatur aus Beispiel 1 verwendet.

Mit dem Strom (1) wurden 8,94 kg/h rohes Produktgas der Cyanwasserstoffherstellung nach dem Andrussow-Verfahren über den Gasventilator (B) in den Kolonnensumpf A zugeführt, das auf das Gewicht bezogen enthielt: HCN: 8,9 %, H₂O: 3,7 %, H₂: 1,3 %, N₂: 76,3 %, O₂: 1,5 %, CO: 5,6 %, CO₂: 1,1 %, CH₄: 0, 4 %. Das eintretende Gas wurde an der Strahlpumpe (B) mit einem umlaufenden Strom (8) von 280 kg/h vermischt. Der Umlaufstrom war dabei so temperiert, dass im Kolonnensumpf (A) bei einem Füllstand von 50% eine Temperatur von 49,8 °C herrschte. Der Zulaufstrom (7) auf die Packung (C) hatte bei 40 kg/h eine Temperatur von 35 °C.

Der Methylthiopropionaldehyd wurde mit einem Durchsatz von 2,976 kg/h über die Zuführung (2) in den Reaktor (G) eingeführt. Er enthielt auf das Gewicht bezogen:
MMP: 96,9 %, H₂O: 2,8 %, MC: 0,2 %. Über die Zuleitung (3) wurden gleichzeitig 0,2 kg/h eines Gemisches aus 99 Gew.% MMP in der oben beschriebenen Zusammensetzung und 1 Gew.% Triethanolamin als Katalysator in den Reaktor (G) eingeführt. Weiterhin wurden 2 kg/h des Sumpfproduktes über den Weg (13) in den Reaktor (G) eingeführt. Der Gesamtstrom (5) bestehend aus den Edukten und dem Kreislaufstrom (6) und dem Produktstrom (13) betrug in der Zuführung zur oberen Packung (E) 42 kg/h bei einer Temperatur von 5,5 °C.

Das molare Eduktverhältnis HCN/MMP entsprach 1. Das Produkt verließ den Kolonnensumpf mit 4,25 kg/h und hatte bezogen auf das Gewicht folgende Zusammensetzung:
MMP-CN: 90,06 %, H₂O: 8,81 %, MMP: 0,75 %, HCN: 0,21 %, MC: 0,01 %. Das Abgas verließ den Kolonnenkopf mit 7,88 kg/h und hatte bezogen auf das Gewicht folgende Zusammensetzung:
   HCN: 0,00 %, MMP: 0,09 %, MC: 0, 10 %, H₂O: 0, %, H2: 1,50 %, N₂: 86, 60 %, O₂: 1,70 %, CO: 6,40 %, CO₂: 1,20 %, CH₄: 0,50 %. Die Gase wurden einer Verbrennungsanlage zugeführt.

## Patentansprüche

1. Verfahren zur Herstellung von 2-Hydroxy-4-(methylthio)buttersäurenitril, bei dem 3-Methylmercapto-propionaldehyd mit Cyanwasserstoff in Gegenwart einer Base als Katalysator in einer Hauptreaktionszone zum Nitril umgesetzt wird und Restmengen an gasförmigem Cyanwasserstoff, welche die Hauptreaktionszone verlassen in einer Absorptions- und Nachreaktionszone enthaltend eine Mischung aus 3-Methylmercaptopropion-aldehyd und Katalysator und wahlweise 2-Hydroxy-4-(methylthio)buttersäurenitril absorbiert und weiter umgesetzt werden.

2. Verfahren gemäß Anspruch 1, **dadurch gekennzeichnet, dass** die in der Absorptions- und Nachreaktionszone enthaltene Mischung zumindest teilweise aus der Hauptreaktionszone stammt.

3. Verfahren gemäß Anspruch 1, **dadurch gekennzeichnet, dass** die Hauptreaktionszone einen Rührreaktor oder Schlaufenreaktor beinhaltet.

4. Verfahren gemäß Anspruch 3, **dadurch gekennzeichnet, dass** die Hauptreaktionszone zusätzlich eine Strahlpumpe beinhaltet.

5. Verfahren gemäß einem der Ansprüche 1 bis 4, **dadurch gekennzeichnet, dass** die Absorptions- und Nachreaktionszone eine Vorrichtung zum In-Kontakt-Bringen eines Gases mit einer Flüssigkeit beinhaltet, vorzugsweise eine Kolonne, insbesondere eine Bodenkolonne, eine Füllkörperkolonne, einen Blasensäulenreaktor, eine Tropfenkolonne oder wahlweise einen Reaktor mit mechanisch gerührtem Behälter, einem Tauchstrahlreaktor oder eine Strahlpumpe beinhaltet.

6. Verfahren gemäß einem der Ansprüche 1 bis 5, **dadurch gekennzeichnet, dass** die Absorptions- und Nachreaktionszone Teil eines Schlaufenreaktors sind.

7. Verfahren gemäß einem der Ansprüche 1 bis 6, **dadurch gekennzeichnet, dass** im Wesentlichen gasförmiger Cyanwasserstoff in die Hauptreaktionszone eingebracht wird, vorzugsweise ein cyanwasserstoffhaltiges Produktgas aus einer Anlage zur Herstellung von Cyanwasserstoff.

8. Verfahren gemäß Anspruch 7 **dadurch gekennzeichnet, dass** der Cyanwasserstoff-Gehalt des eingesetzten Gases von 1 bis 99 Gew.%, vorzugsweise von 5 bis 75 Gew.%, besonders bevorzugt von 6-22 Gew.% beträgt.

9. Verfahren gemäß einem der Ansprüche 1 bis 8 **dadurch gekennzeichnet, dass** als Katalysator niedermolekulare oder heterogene Amine, Lösungen von anorganischen Basen oder Mischungen aus Säuren und niedermolekularen Aminen verwendet werden.

10. Verfahren gemäß Anspruch 9 **dadurch gekennzeichnet, dass** als niedermolekulare Amine Tri-(C₁-C₁₂-alkyl)-amine, vorzugsweise Triethylamin oder Triisopropanolamin, Dialkylaralkylamine, vorzugsweise Dimethylbenzylamin, Dialkylarylamine, vorzugsweise N,N-Dimethylanilin, heterozyklische Amine, vorzugsweise Nicotinamid, Imidazol, Benzimidazol, 2-Fluorpyridin, 4-Dimethylaminopyridin, Picolin oder Pyrazin, verwendet werden.

11. Verfahren gemäß Anspruch 9, **dadurch gekennzeichnet, dass** heterogene Amine der allgemeinen Formel 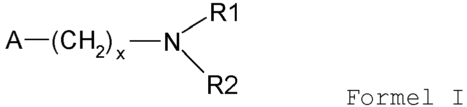 oder Polyvinylpyridin verwendet werden, wobei
R₁ und R₂ Wasserstoff, Alkyl mit Kettenlängen zwischen C₁ und C₁₂, Aryl oder Heteroaryl sind;
R₁ kann verschieden von R₂ sein
X ist eine Zahl zwischen 0 und 6, und
A ist ein natürliches oder synthetisches Harz, vorzugsweise ein Polystyrol.

12. Verfahren gemäß Anspruch 11, **dadurch gekennzeichnet dass** der Katalysator gemäß Formel I eine Polymer-gebundene Base ausgewählt aus der Gruppe der homologen Dialkylaminoalkylpolystyrole oder Dialkylaminomakroretikulären Harze ist.

13. Verfahren gemäß Anspruch 12, **dadurch gekennzeichnet, dass** der Katalysator gemäß Formel I Diethylaminoethylpolystyrol, Diethylaminomethylpolystyrol, Dimethylaminomethylpolystyrol, Diethylaminomethylmakroretikuläres Harz oder Dimethylaminoethylpolystyrol ist.

14. Verfahren gemäß Anspruch 9 **dadurch gekennzeichnet, dass** als anorganische Base Alkalihydroxid, vorzugsweise NaOH oder KOH, Alkalicyanid, vorzugsweise NaCN oder KCN, Alkalicarbonat, vorzugsweise Na₂CO₃ oder K₂CO₃, oder Alkalihydrogencarbonat, vorzugsweise NaHCO₃ oder KHCO₃, alleine oder in gemischter Form verwendet werden.

15. Verfahren gemäß Anspruch 9, **dadurch gekennzeichnet, dass** als Säure in den Mischungen aus Säuren und niedermolekularen Aminen organischen Säuren, vorzugsweise, kurzkettigen Fettsäuren, insbesondere Essigsäure, Ameisensäure, Zitronensäure, oder organische Sulfonsäuren, vorzugsweise Trifluormethansulfonsäure oder Mineralsäuren, vorzugsweise Schwefelsäure oder Phosphorsäure verwendet werden.

16. Verfahren gemäß einem der Ansprüche 1 bis 15, **dadurch gekennzeichnet, dass** ein pH-Wert von 4,5 bis 6,0, vorzugsweise 5,0-5,5 gemessen mit einer pH-Elektrode bei 23°C und einem Wassergehalt von 2 bis 14 Gew.% in der Hauptreaktionszone und der Absorptions-und Nachreaktionszone eingestellt wird.

17. Verfahren gemäß einem der Ansprüche 1 bis 16, **dadurch gekennzeichnet, dass** die Hauptreaktionszone bei einer Temperatur von 20°C bis 80°C, vorzugsweise von 30°C bis 70°C, besonders bevorzugt von 35°C bis 65°C betrieben wird.

18. Verfahren gemäß einem der Ansprüche 1 bis 17, **dadurch gekennzeichnet, dass** die Absorptions- und Nachreaktionszone bei einer Temperatur von 0°C bis 30°C, vorzugsweise von 4°C bis 15°C betrieben wird.

19. Verfahren gemäß einem der Ansprüche 1 bis 18, **dadurch gekennzeichnet, dass** eine weitere Nachreaktionszone vor der Produktaustragsstelle von MMP-Cyanhydrin zur Vervollständigung der Umsetzung von HCN und MMP zum MMP-Cyanhydrin bei einer Temperatur von 20°C bis 80°C, vorzugsweise von 40°C bis 70°C , besonders bevorzugt von 45°C bis 65°C betrieben wird.

20. Verfahren gemäß einem der Ansprüche 1 bis 19 **dadurch gekennzeichnet, dass** unter einem Absolutdruck von 0,9 bis 5 bar, vorzugsweise von 1,0 bis 3 bar, besonders bevorzugt von 1 bis 1,5 bar gearbeitet wird.

21. Verfahren gemäß einem der Ansprüche 1 bis 20, **dadurch gekennzeichnet, dass** das molare Verhältnis von Blausäure zu 3-(Methylthio)propanal von 0,98 bis 1,03, bevorzugt von 0,99 bis 1,01 ist.

22. Verfahren gemäß einem der Ansprüche 1 bis 21, **dadurch gekennzeichnet, dass** ein Gewichtsverhältnis von Katalysator zu 3-(Methylthio)propanal von 0,00005 bis 0,002 vorzugsweise von 0,0001 bis 0,001 verwendet wird.

23. Verfahren gemäß einem der Ansprüche 1 bis 22, **dadurch gekennzeichnet, dass** das Verfahren im Batch, halbkontinuierlich oder kontinuierlich durchgeführt wird.

24. Verwendung eines gemäß einem der Ansprüche 1 bis 23 hergestellten MMP-Cyanhydrin-haltigen Reaktionsproduktes zur Herstellung von D,L-Methionin oder von 2-Hydroxy-4-methylthiobuttersäure.

## Claims

1. Method for producing 2-hydroxy-4-(methylthio)butyronitrile, in which 3-methylmercaptopropionaldehyde is reacted with hydrogen cyanide in the presence of a base as catalyst in a main reaction zone to form the nitrile and residual amounts of gaseous hydrogen cyanide which leave the main reaction zone are absorbed in an absorption and post-reaction zone containing a mixture of 3-methylmercaptopropionaldehyde and catalyst and optionally 2-hydroxy-4-(methylthio)butyronitrile and are further reacted.

2. Method according to Claim 1, **characterized in that** the mixture contained in the absorption and post-reaction zone originates at least partially from the main reaction zone.

3. Method according to Claim 1, **characterized in that** the main reaction zone contains a stirred reactor or loop reactor.

4. Method according to Claim 3, **characterized in that** the main reaction zone additionally contains a jet pump.

5. Method according to any one of Claims 1 to 4, **characterized in that** the absorption and post-reaction zone contains a device for contacting a gas with a liquid, preferably a column, in particular a tray column, a packed-bed column, a bubble-column reactor, a droplet column or optionally a reactor having a mechanically agitated container, a submerged jet reactor or a jet pump.

6. Method according to any one of Claims 1 to 5, **characterized in that** the absorption and post-reaction zones are part of a loop reactor.

7. Method according to any one of Claims 1 to 6, **characterized in that** substantially gaseous hydrogen cyanide is introduced into the main reaction zone, preferably a hydrogen-cyanide-containing product gas from a plant for producing hydrogen cyanide.

8. Method according to Claim 7, **characterized in that** the hydrogen cyanide content of the gas used is 1 to 99% by weight, preferably 5 to 75% by weight, particularly preferably 6-22% by weight.

9. Method according to any one of Claims 1 to 8, **characterized in that** the catalyst used is low-molecular-weight or heterogeneous amines, solutions of inorganic bases, or mixtures of acids and low-molecular-weight amines.

10. Method according to Claim 9, **characterized in that** the low-molecular-weight amines are tri-(C₁-C₁₂-alkyl)amines, preferably triethylamine or triisopropanolamine, dialkylaralkylamines, preferably dimethylbenzylamine, dialkylarylamines, preferably N,N-dimethylaniline, heterocyclic amines, preferably nicotinamide, imidazole, benzimidazole, 2-fluoropyridine, 4-dimethylaminopyridine, picoline or pyrazine.

11. Method according to Claim 9, **characterized in that** heterogeneous amines of the general formula 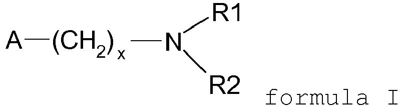 or polyvinylpyridine are used, wherein
R₁ and R₂ are hydrogen, alkyl having chain lengths between C₁ and C₁₂, aryl or heteroaryl;
R₁ can be different from R₂
X is a number between 0 and 6, and
A is a natural or synthetic resin, preferably a polystyrene.

12. Method according to Claim 11, **characterized in that** the catalyst according to formula I is a polymer-bound base selected from the group of homologous dialkylaminoalkylpolystyrenes or dialkylaminomacroreticular resins.

13. Method according to Claim 12, **characterized in that** the catalyst according to formula I is diethylaminoethylpolystyrene, diethylaminomethylpolystyrene, dimethylaminomethylpolystyrene, diethylaminomethylmacroreticular resin or dimethylaminoethylpolystyrene.

14. Method according to Claim 9, **characterized in that** the inorganic base used is alkali metal hydroxide, preferably NaOH or KOH, alkali metal cyanide, preferably NaCN or KCN, alkali metal carbonate, preferably Na₂CO₃ or K₂CO₃, or alkali metal hydrogencarbonate, preferably NaHCO₃ or KHCO₃, alone or in mixed form.

15. Method according to Claim 9, **characterized in that** the acid used in the mixtures of acids and low-molecular-weight amines is organic acids, preferably short-chain fatty acids, in particular acetic acid, formic acid, citric acid, or organic sulfonic acids, preferably trifluoromethanesulfonic acid or mineral acids, preferably sulfuric acid or phosphoric acid.

16. Method according to any one of Claims 1 to 15, **characterized in that** a pH of 4.5 to 6.0 is set, preferably 5.0-5.5, measured using a pH electrode at 23°C and a water content of 2 to 14% by weight in the main reaction zone and the absorption and post-reaction zone.

17. Method according to any one of Claims 1 to 16, **characterized in that** the main reaction zone is operated at a temperature of 20°C to 80°C, preferably 30°C to 70°C, particularly preferably 35°C to 65°C.

18. Method according to any one of Claims 1 to 17, **characterized in that** the absorption and post-reaction zone is operated at a temperature of 0°C to 30°C, preferably 4°C to 15°C.

19. Method according to any one of Claims 1 to 18, **characterized in that** a further post-reaction zone is operated at a temperature of 20°C to 80°C, preferably 40°C to 70°C, particularly preferably 45°C to 65°C, upstream of the MMP-cyanohydrin product discharge site for completing the reaction of HCN and MMP to form the MMP-cyanohydrin.

20. Method according to any one of Claims 1 to 19, **characterized in that** an absolute pressure of 0.9 to 5 bar, preferably 1.0 to 3 bar, particularly preferably 1 to 1.5 bar, is employed.

21. Method according to any one of Claims 1 to 20, **characterized in that** the molar ratio of prussic acid to 3-(methylthio)propanal is 0.98 to 1.03, preferably 0.99 to 1.01.

22. Method according to any one of Claims 1 to 21, **characterized in that** a weight ratio of catalyst to 3-(methylthio)propanal of 0.00005 to 0.002, preferably 0.0001 to 0.001, is used.

23. Method according to any of Claims 1 to 22, **characterized in that** the method is carried out batchwise, semicontinuously, or continuously.

24. Use of a reaction product containing MMP-cyanohydrin produced according to any one of Claims 1 to 23 for producing D,L-methionine or 2-hydroxy-4-methylthiobutyric acid.

## Revendications

1. Procédé de fabrication d'un nitrile de l'acide 2-hydroxy-4-(méthylthio)butyrique, selon lequel du 3-méthylmercapto-propionaldéhyde est transformé en le nitrile avec du cyanure d'hydrogène en présence d'une base en tant que catalyseur dans une zone de réaction principale, et les quantités résiduelles de cyanure d'hydrogène gazeux qui quittent la zone de réaction principale sont absorbées dans une zone d'absorption et de post-réaction contenant un mélange de 3-méthylmercaptopropionaldéhyde et de catalyseur et éventuellement du nitrile de l'acide 2-hydroxy-4-(méthylthio)butyrique, et davantage mises en réaction.

2. Procédé selon la revendication 1, **caractérisé en ce que** le mélange contenu dans la zone d'absorption et de post-réaction provient au moins en partie de la zone de réaction principale.

3. Procédé selon la revendication 1, **caractérisé en ce que** la zone de réaction principale contient un réacteur agité ou un réacteur à boucle.

4. Procédé selon la revendication 3, **caractérisé en ce que** la zone de réaction principale contient en outre une pompe à jet.

5. Procédé selon l'une quelconque des revendications 1 à 4, **caractérisé en ce que** la zone d'absorption et de post-réaction contient un dispositif pour la mise en contact d'un gaz avec un liquide, de préférence une colonne, notamment une colonne à plateaux, une colonne à corps de remplissage, un réacteur à colonne à bulles, une colonne à gouttes ou éventuellement un réacteur à contenant agité mécaniquement, un réacteur à jet noyé ou une pompe à jet.

6. Procédé selon l'une quelconque des revendications 1 à 5, **caractérisé en ce que** la zone d'absorption et de post-réaction fait partie d'un réacteur à boucle.

7. Procédé selon l'une quelconque des revendications 1 à 6, **caractérisé en ce que** du cyanure d'hydrogène essentiellement gazeux est introduit dans la zone de réaction principale, de préférence un produit gazeux contenant du cyanure d'hydrogène issu d'une unité de fabrication de cyanure d'hydrogène.

8. Procédé selon la revendication 7, **caractérisé en ce que** la teneur en cyanure d'hydrogène du gaz utilisé est de 1 à 99 % en poids, de préférence de 5 à 75 % en poids, de manière particulièrement préférée de 6 à 22 % en poids.

9. Procédé selon l'une quelconque des revendications 1 à 8, **caractérisé en ce que** des amines de faible poids moléculaire ou hétérogènes, des solutions de bases inorganiques ou des mélanges d'acides et d'amines de faible poids moléculaire sont utilisés en tant que catalyseur.

10. Procédé selon la revendication 9, **caractérisé en ce que** des tri-(alkyle en C₁-C₁₂) -amines, de préférence la triéthylamine ou la triisopropylamine, des dialkylaralkylamines, de préférence la diméthylbenzylamine, des dialkylarylamines, de préférence la N,N-diméthylaniline, des amines hétérocycliques, de préférence le nicotinamide, l'imidazole, le benzimidazole, la 2-fluoropyridine, la 4-diméthylaminopyridine, la picoline ou la pyrazine sont utilisées en tant qu'amines de faible poids moléculaire.

11. Procédé selon la revendication 9, **caractérisé en ce que** des amines hétérogènes de formule générale 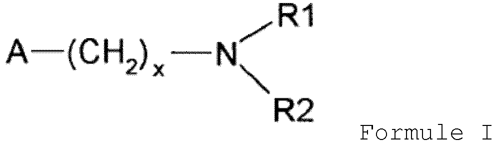 ou la polyvinylpyridine sont utilisées,
R₁ et R₂ représentant hydrogène, alkyle de longueurs de chaîne comprises entre C₁ et C₁₂, aryle ou hétéroaryle ;
R₁ pouvant être différent de R₂,
X représentant un nombre compris entre 0 et 6, et
A étant une résine naturelle ou synthétique, de préférence un polystyrène.

12. Procédé selon la revendication 11, **caractérisé en ce que** le catalyseur selon la formule I est une base reliée à un polymère choisie dans le groupe des dialkylaminoalkylpolystyrènes homologues ou des résines dialkylamino macroréticulaires.

13. Procédé selon la revendication 12, **caractérisé en ce que** le catalyseur selon la formule I est le diéthylaminoéthylpolystyrène, le diéthylaminométhylpolystyrène, le diméthylaminométhylpolystyrène, une résine diéthylaminométhyle macroréticulaire ou le diméthylaminoéthylpolystyrène.

14. Procédé selon la revendication 9, **caractérisé en ce qu'**un hydroxyde alcalin, de préférence NaOH ou KOH, un cyanure alcalin, de préférence NaCN ou KCN, un carbonate alcalin, de préférence Na₂CO₃ ou K₂CO₃, ou un hydrogénocarbonate alcalin, de préférence NaHCO₃ ou KHCO₃, sont utilisés seuls ou sous forme mélangée en tant que base inorganique.

15. Procédé selon la revendication 9, **caractérisé en ce que** des acides organiques, de préférence des acides gras à chaîne courte, notamment l'acide acétique, l'acide formique, l'acide citrique, ou des acides sulfoniques organiques, de préférence l'acide trifluorométhanesulfonique, ou des acides minéraux, de préférence l'acide sulfurique ou l'acide phosphorique, sont utilisés en tant qu'acide dans les mélanges d'acides et d'amines de faible poids moléculaire.

16. Procédé selon l'une quelconque des revendications 1 à 15, **caractérisé en ce qu'**un pH de 4,5 à 6,0, de préférence de 5,0 à 5,5, mesuré avec une électrode de pH à 23 °C et à une teneur en eau de 2 à 14 % en poids est ajusté dans la zone de réaction principale et dans la zone d'absorption et de post-réaction.

17. Procédé selon l'une quelconque des revendications 1 à 16, **caractérisé en ce que** la zone de réaction principale est exploitée à une température de 20 °C à 80 °C, de préférence de 30 °C à 70 °C, de manière particulièrement préférée de 35 °C à 65 °C.

18. Procédé selon l'une quelconque des revendications 1 à 17, **caractérisé en ce que** la zone d'absorption et de post-réaction est exploitée à une température de 0 °C à 30 °C, de préférence de 4 °C à 15 °C.

19. Procédé selon l'une quelconque des revendications 1 à 18, **caractérisé en ce qu'**une zone de post-réaction supplémentaire avant l'emplacement de déchargement du produit de MMP-cyanhydrine pour la complétion de la transformation d'HCN et de MMP en MMP-cyanhydrine est exploitée à une température de 20 °C à 80 °C, de préférence de 40 °C à 70 °C, de manière particulièrement préférée de 45 °C à 65 °C.

20. Procédé selon l'une quelconque des revendications 1 à 19, **caractérisé en ce qu'**il est réalisé sous une pression absolue de 0,9 à 5 bar, de préférence de 1,0 à 3 bar, de manière particulièrement préférée de 1 à 1,5 bar.

21. Procédé selon l'une quelconque des revendications 1 à 20, **caractérisé en ce que** le rapport molaire entre l'acide cyanhydrique et le 3-(méthylthio)propanal est de 0,98 à 1,03, de préférence de 0,99 à 1,01.

22. Procédé selon l'une quelconque des revendications 1 à 21, **caractérisé en ce qu'**un rapport en poids entre le catalyseur et le 3-(méthylthio)propanal de 0,00005 à 0,002, de préférence de 0,0001 à 0,001 est utilisé.

23. Procédé selon l'une quelconque des revendications 1 à 22, **caractérisé en ce que** le procédé est réalisé de manière discontinue, semi-continue ou continue.

24. Utilisation d'un produit de réaction contenant de la MMP-cyanhydrine fabriqué selon l'une quelconque des revendications 1 à 23 pour la fabrication de D,L-méthionine ou d'acide 2-hydroxy-4-méthylthiobutyrique.
